# EUROPEAN PATENT APPLICATION

(11) **EP 4 623 840 A1**
(43) Date of publication of application: **01.10.2025**
(21) Application number: 23893804.7
(22) Date of filing: 21.11.2023
(51) Int. Cl.: A61B 17/068, A61B 17/072, A61B 17/115, F16H 19/04, H02K 7/116, H02K 7/06

(54) **EMERGENCY APPARATUS AND SURGICAL INSTRUMENT**

(30) Priority: 23.11.2022 CN 202211474601
(71) Applicant: Touchstone International Medical Science Co., Ltd., Suzhou, Jiangsu 215123 (CN)
(72) Inventor: CHEN, Wangdong, Suzhou, Jiangsu 215123 (CN); WANG, Yeting, Suzhou, Jiangsu 215123 (CN); HUANG, Bin, Suzhou, Jiangsu 215123 (CN); DAI, Xiahong, Suzhou, Jiangsu 215123 (CN)
(74) Representative: Winter, Brandl - Partnerschaft mbB
(86) International application number: PCT/CN2023/132808
(87) International publication number: WO 2024/109714

(57) **Abstract**

Provided are an emergency apparatus and a surgical instrument. The surgical instrument comprises a transmission mechanism (6), a connection mechanism (4) connected to the transmission mechanism (6), and an electric mechanism (5), wherein the connection mechanism (4) comprises a first gear (41), and the connection mechanism (4) is disengaged from the electric mechanism (5) by means of the disengagement of the first gear (41) from the electric mechanism (5). The emergency apparatus comprises: a movable shaft (43) movably arranged through the first gear (41). When the movable shaft (43) and the first gear (41) are in a first coordination state, the first gear (41) can rotate around the movable shaft (43); when the movable shaft (43) and the first gear (41) are in a second coordination state, the rotation of the first gear (41) around the movable shaft (43) is limited, and the first gear (41) can be driven to move by means of the movement of the movable shaft (43) so as to be disengaged from the electric mechanism (5), wherein the movement direction of the movable shaft (43) is consistent with that of the first gear (41).

## Description

### TECHNICAL FIELD

The present application relates to the technical field of medical instruments, and in particular, to an emergency apparatus and a surgical instrument.

### BACKGROUND

In some conventional surgical instruments, an operation performed by surgeons is received at a proximal end by a firing mechanism, which drives a transmission shaft to move toward the distal end, thereby actuating an operating mechanism located at a distal end to perform corresponding surgical procedures. One example of such surgical instruments is a surgical stapler. Existing surgical stapler includes a stapler body, a firing mechanism (e.g., a firing handle) movably connected to the stapler body, and an end effector coupled to the stapler body. The stapler body includes a driving assembly. The end effector includes an anvil and a staple cartridge that are arranged opposite each other. During surgery, firstly, tissue is disposed between the anvil and the staple cartridge, the distance therebetween is then adjusted to gradually clamp the tissue, an operation received by the firing mechanism subsequently drives the driving assembly to move toward the distal end, enabling staples to form against the anvil. A stapling of tissue is thus completed.

During surgery, typically, surgeons are required to apply a significant amount of force to the firing mechanism, which presents inconvenience. To address this, a motor-driven surgical instrument has been developed. Once a motor thereof is activated, an output shaft of the motor rotates in a specific direction, so as to move the transmission mechanism toward the distal end. After the completion of one surgical procedure, the output shaft of the motor can be reversed, which is referred as an electric retraction function, and in response to which the transmission mechanism moves toward the proximal end to reset the entire instrument. However, malfunctions occurring in the motor, the associated mechanical/electronic components may impede the electric retraction function, preventing instruments from resetting, locking a jaw opening of the end effector, thus compromising surgical procedures.

### SUMMARY

To address the aforementioned issues in prior art, the objective of the present application is to provide an emergency apparatus and a surgical instrument. In an event that an electric mechanism fails to reset a transmission mechanism after the completion the firing, an emergency apparatus is suitable for manually resetting the transmission mechanism.

**In** some embodiments of the present application, an emergency apparatus for a surgical instrument is provided. The surgical instrument includes: a transmission mechanism, driven to move toward or away from an end effector, of the surgical instrument; a connection mechanism connected to the transmission mechanism; and an electric mechanism, connected to the connection mechanism, configured to move the connection mechanism, thereby driving the transmission mechanism to move. The connection mechanism includes a first gear. The connection mechanism is configured to disengage from the electric mechanism in response to the disengagement of the first gear from the electric mechanism. The emergency apparatus includes a movable shaft movably disposed through the first gear, when the movable shaft and the first gear are in a first meshing state, the first gear is rotatable around the movable shaft; when the movable shaft and the first gear are in a second meshing state, the first gear is prevented from rotating around the movable shaft, and the first gear is configured to move in response to a movement of the movable shaft, thereby being disengaged from the electric mechanism, where the movement direction of the movable shaft is consistent with the movement direction of the first gear.

**In** some embodiments, when the movable shaft and the first gear are located at a first relative position, they are in the first meshing state; when the movable shaft and the first gear are located at a second relative position, they are in the second meshing state; where the movable shaft and the first gear convert from the first relative position to the second relative position in response to a movement of the movable shaft in a first direction. The first direction is perpendicular to a direction in which the transmission mechanism moves toward or away from the end effector of the surgical instrument.

In some embodiments, the movable shaft includes a first mating portion, a second mating portion and a third mating portion, while the first gear includes a fourth mating portion and a fifth mating portion. When the movable shaft and the first gear are in the first meshing state, the first mating portion cooperates with the fourth mating portion to allow the first gear to rotate around the movable shaft. When the movable shaft and the first gear are in the second meshing state, the second mating portion cooperates with the fourth mating portion to prevent the first gear from rotating around the movable shaft. When the movable shaft is driven to move, the third mating portion cooperates with the fifth mating portion to drive the first gear to move, thereby disengaging the first gear from the electric mechanism.

In some embodiments, the first gear includes a shaft hole through which the movable shaft is disposed. The movable shaft is provided with a first threaded portion. The emergency apparatus further includes a driving member having a second threaded portion for cooperating with the first threaded portion. When the driving member rotates, the movable shaft is driven to move.

In some embodiments, both ends of the movable shaft protrude beyond end faces at two sides of the first gear, respectively, and the first threaded portion is disposed at a first end of the movable shaft, the third mating portion being disposed at a second end thereof. The third mating portion includes an outer diameter greater than an inner diameter of the shaft hole of the first gear.

In some embodiments, the first mating portion of the movable shaft is located between the first threaded portion and the second mating portion and includes an outer diameter less than or equal to the inner diameter of the shaft hole of the first gear.

In some embodiments, in an event that the movable shaft moves in response to the rotation of the driving member after the first gear has disengaged from the electric mechanism, the first gear rotates in response to the movement of the movable shaft due to a cooperation formed between the second mating portion and the fourth mating portion.

In some embodiments, the emergency apparatus further includes an elastic member configured to apply a biasing force to the first gear in a second direction opposite to the first direction.

In some embodiments, the first threaded portion includes an external thread distributed on an outer surface of the movable shaft. The driving member is at least partially sleeved over the first threaded portion. The second threaded portion includes an internal thread distributed on an inner surface of the driving member.

In some embodiments, the emergency apparatus further includes a first operating lever, the driving member is provided at a first end of the first operating lever.

In some embodiments, the first end of the first operating lever is provided with an mating hole or an mating groove. The driving member is at least partially embedded within said mating hole or mating groove.

In some embodiments, the outer surface of the driving member cooperates with an inner surface of the mating hole or the mating groove to form a non-rotatable connection.

In some embodiments, the driving member is detachably connected to the first end of the first operating lever.

In some embodiments, the emergency apparatus further includes a second operating lever connected to a second end of the first operating lever.

In some embodiments, the emergency apparatus further includes an outer frame, the first gear and the movable shaft are at least partially housed within the outer frame, the outer frame includes a receiving slot disposed on one side thereof, the second operating lever is at least partially housed within the receiving slot.

In some embodiments, the electric mechanism includes a motor and a motor gear assembly, an output shaft of the motor is configured to drive the motor gear assembly to rotate. The motor gear assembly is at least partially located on one side of the first gear along the second direction and includes a second gear, the second direction opposites to the first direction. When the electric mechanism is connected to the connection mechanism, the first gear meshes with the second gear, with the output shaft of motor parallel to the first direction.

In some embodiments, the motor gear assembly includes a first motor gear and a second motor gear that have axes arranged crossly. The output shaft of motor could be parallel to the first direction, or could be oriented at a first angle.

The present application further provides a surgical instrument including any of the aforementioned emergency apparatuses.

The emergency apparatus and surgical instrument provided in this application have the following advantages:

In an event that the electric mechanism fails to reset the transmission mechanism after the completion of instrument firing, the movable shaft is configured to drive the first gear to disengage from the electric mechanism, further of rotating the first gear to reset the transmission mechanism, thereby achieving an emergency manual reset. This enables a convenient operation under emergency, ensuring uninterrupted surgical procedures.

Furthermore, since the driving member provided at the first end of the first operating lever includes the second threaded portion cooperating with the first threaded portion of the movable shaft, the movable shaft moves in response to the rotation of the driving member. Such configuration guarantees a maintained connection between the first operating lever and movable shaft during an operation of the emergency apparatus, preventing the first lever form disengaging form the movable shaft, thereby facilitating the emergency manual reset. Such configuration further avoiding potential operational failures on emergency manual reset that might exacerbate surgeons' stress in emergency scenarios, thereby safeguarding surgical outcomes.

### BRIEF DESCRIPTION OF THE DRAWINGS

Further features, objectives, and advantages of the present application will become more apparent from the detailed description of non-limiting embodiments with reference to the accompanying drawings.
Figs. 1 illustrates a schematic view of the emergency apparatus according to the first embodiment of the present application, where the emergency apparatus cooperates with other instrument components.
Figs. 2 illustrates a schematic view of the emergency apparatus according to the first embodiment of the present application, where the emergency apparatus cooperates with other instrument components.
Figs. 3 illustrates a schematic view of the emergency apparatus according to the first embodiment of the present application, where the emergency apparatus cooperates with other instrument components.
Fig. 4 illustrates a structural schematic view of the outer frame according to the first embodiment of the present application.
Fig. 5 illustrates a schematic view of the emergency apparatus according to the first embodiment of the present application, where the emergency apparatus cooperates with other instrument components with the outer frame omitted.
Fig. 6 illustrates a schematic view of the emergency apparatus according to the first embodiment of the present application, where the emergency apparatus cooperates with other instrument components with the outer frame and the inner frame omitted.
Fig. 7 illustrates a front view of the emergency apparatus according to the first embodiment of the present application, where the emergency apparatus cooperates with other instrument components with the outer frame and the inner frame omitted.
Fig. 8 illustrates a schematic view of the movable shaft according to the first embodiment of the present application, where the movable shaft cooperates with other instrument components.
Fig. 9 illustrates a structural schematic view of the operating mechanism according to the first embodiment of the present application, where the operating mechanism cooperates with the driving member.
Fig. 10 illustrates a structural schematic view of the movable shaft according to the first embodiment of the present application, where the movable shaft cooperates with the first gear.
Figs. 11 illustrates a schematic view of the movable shaft according to the first embodiment of the present application, where the movable shaft disengages from the first gear.
Figs. 12 illustrates a schematic view of the movable shaft according to the first embodiment of the present application, where the movable shaft disengages from the first gear.
Fig. 13 illustrates a schematic view of the emergency apparatus according to the first embodiment of the present application, where the first gear has disengaged from the electric mechanism, and the emergency apparatus cooperates with other instrument components.
Fig. 14 illustrates a schematic view of the emergency apparatus according to the second embodiment of the present application, where the emergency apparatus cooperates with other instrument components.
Fig. 15 illustrates a schematic view of the emergency apparatus according to the second embodiment of the present application, where the emergency apparatus cooperates with other instrument components with the outer frame omitted.
Fig. 16 illustrates a front view of the emergency apparatus according to the second embodiment of the present application, where the emergency apparatus cooperates with other instrument components with the outer frame and the inner frame omitted.
Fig. 17 illustrates a schematic view of the emergency apparatus according to the second embodiment of the present application, where the emergency apparatus cooperates with other instrument components with the outer frame and the inner frame omitted.
Fig. 18 illustrates a bottom view of the movable shaft according to the second embodiment of the present application, where the movable shaft cooperates with other instrument components.
Fig. 19 illustrates a schematic view of the emergency apparatus according to the second embodiment of the present application, where the first gear cooperates with the second motor gear, and the emergency apparatus cooperates with other instrument components.
Fig. 20 illustrates a schematic view of the emergency apparatus according to the second embodiment of the present application, where the first gear has disengaged from the second motor gear, and the emergency apparatus cooperates with other instrument components.
Fig. 21 illustrates a front view of the emergency apparatus according to the third embodiment of the present application, where the emergency apparatus cooperates with other instrument components.
Fig. 22 illustrates a schematic view of the emergency apparatus according to the third embodiment of the present application, where the emergency apparatus cooperates with other instrument components.
Fig. 23 illustrates a front view of the emergency apparatus according to the third embodiment of the present application, where the first gear has disengaged from the electric mechanism, and the emergency apparatus cooperates with other instrument components.
Fig. 24 illustrates a structural schematic view of the operating mechanism according to the third embodiment of the present application.

### Reference Numerals:

1 Outer frame
12 Receiving slot
2 Operating mechanism
21 First operating lever
211 mating hole
212 mating groove
22 Second operating lever
23 Pivot shaft
3 Driving member
31 Second threaded portion
32 Mating surface
4 Connection mechanism
41 First gear
411 Fourth mating portion
412 Fifth mating portion
413 Shaft hole
42 Elastic member
43 Movable shaft
431 First threaded portion
432 Second mating portion
433 Third mating portion
434 First mating portion
5 Electric mechanism
51 Motor
52 Second gear
53 Motor gear assembly
531 First motor gear
532 Second motor gear
5321 First teeth portion
5322 Second teeth portion
6 Transmission mechanism
61 Teeth surface
7 Inner frame
71 Limiting portion

### DETAILED DESCRIPTIONS OF THE EMBODIMENTS

Exemplary embodiments will now be described more fully with reference to the accompanying drawings. However, the exemplary embodiments may be implemented in various forms and should not be construed as limited to the embodiments set forth herein. Rather, these embodiments are provided to make this application thorough and complete, and to fully convey the concepts of the exemplary embodiments to those skilled in the art. Throughout the drawings, like reference numerals designate identical or similar structures, and repetitive descriptions thereof will be omitted. The terms 'or' and 'either' in the specification may indicate 'and' or 'alternatively'. Although terms such as 'upper', 'lower', and 'between' may be used herein to describe various exemplary features and elements of the present application, these terms are used merely for convenience, e.g., based on the orientation shown in the accompanying drawings. Nothing in this specification should be interpreted as requiring a specific three-dimensional orientation to fall within the scope of the present application. While terms such as "first" to 'fifth' are used to denote certain features, they are merely indicative and do not limit the quantity or importance of the specific features.

The present application provides an emergency apparatus for a surgical instrument and a surgical instrument including said apparatus. The surgical instrument includes an instrument body and an end effector located at a distal end of the instrument body. The instrument body includes: a transmission mechanism that can be driven to move toward or away from the end effector of the surgical instrument; a connection mechanism connected to the transmission mechanism; and an electric mechanism connected to the connection mechanism and configured to drive the connection mechanism to move, thereby driving the transmission mechanism to move. The connection mechanism includes a first gear. The connection mechanism disengages from the electric mechanism in response to the disengagement of the first gear from the electric mechanism. The surgical instrument may be, for example, a surgical stapler or other types of surgical instruments.

The emergency apparatus includes: a movable shaft movably disposed through the first gear, when the movable shaft and the first gear are in a first meshing state, the first gear is rotatable around the movable shaft; when the movable shaft and the first gear are in a second meshing state, the first gear is prevented from rotating around the movable shaft, and the first gear is driven to rotate by the rotation of the movable shaft. The first gear is configured to move in response to a movement of the movable shaft, thereby being disengaged from the electric mechanism, and the movement direction of the movable shaft is consistent with the movement direction of the first gear. In an event that the electric mechanism fails to reset the transmission mechanism after the firing of instrument, the movable shaft is configured to drive the first gear to disengage from the electric mechanism, and drive the first gear to rotate to reset the transmission mechanism, thereby achieving an emergency manual reset.

The structure of the emergency apparatus and the surgical instrument according to specific embodiments will be described in detail with reference to the drawings. It should be understood that these embodiments do not limit the protection scope of this application.

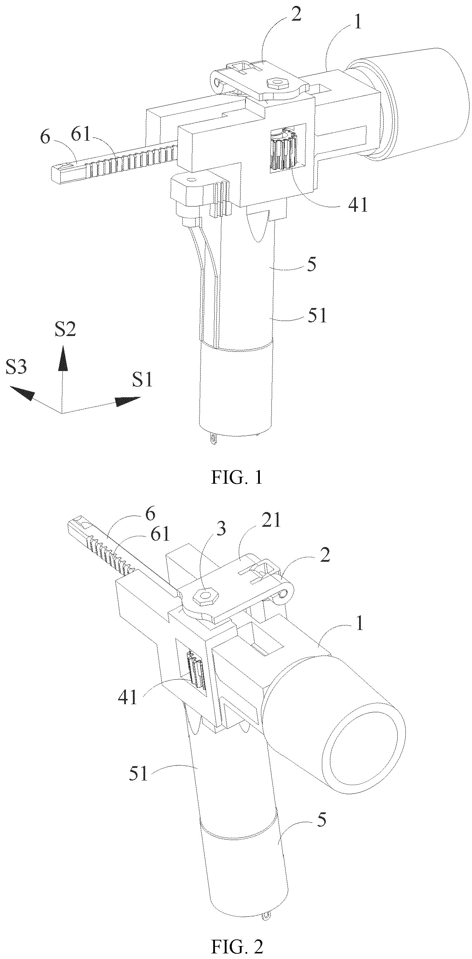

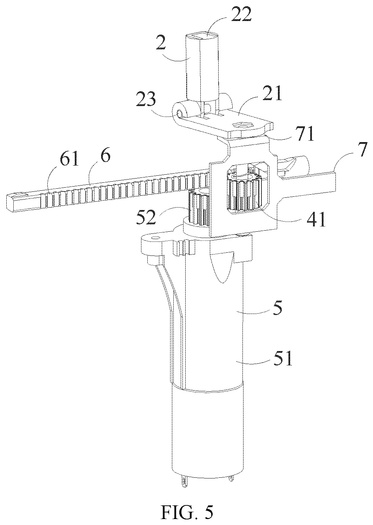

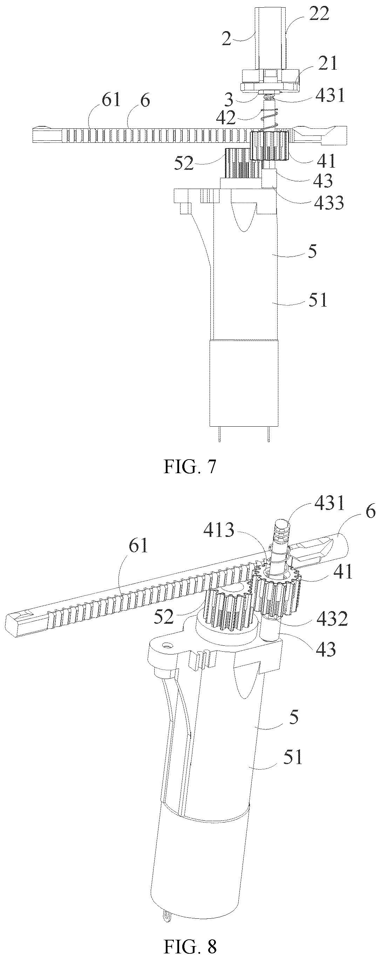

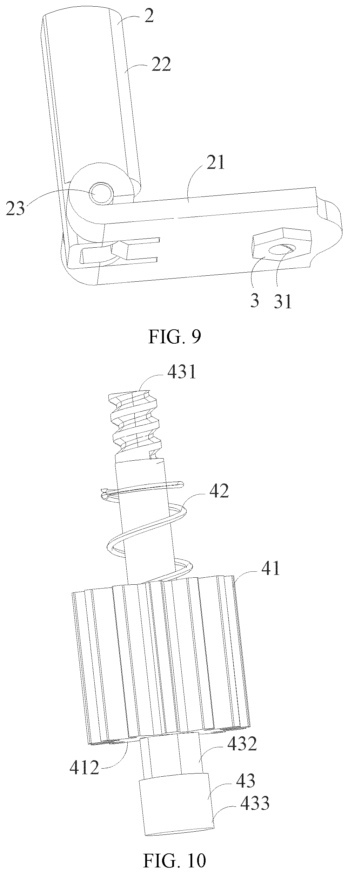

In the first embodiment of the present application, the surgical instrument comprises an instrument body and an end effector located at the distal end of the instrument body, with an emergency apparatus disposed at the instrument body. This embodiment is illustrated taking a surgical stapler as an example, where the end effector refers to the end effector performing stapling/cutting actions. Figs. 1-13 illustrate the emergency apparatus according to the first embodiment, where the emergency apparatus cooperates with other instrument components including, but not limited to, a transmission mechanism 6, a connection mechanism 4, and an electric mechanism 5.

As shown in Figs. 1-8, the instrument body includes a transmission mechanism 6 that can be driven to move toward or away from the end effector of the surgical instrument, a connection mechanism 4 connected to the transmission mechanism 6, and an electric mechanism 5 connected to the connection mechanism 4 and configured to drive the connection mechanism 4 to move, thereby driving the transmission mechanism 6 to move. The transmission mechanism 6 is provided with a teeth surface 61 on its side facing the connection mechanism 4. The connection mechanism 4 includes a first gear 41 meshing with the teeth surface 61 of the transmission mechanism 6. The connection mechanism 4 is configured to disengage from the electric mechanism 5 in response to a disengagement of the first gear 41 from the electric mechanism 5. The electric mechanism 5 comprises a motor 51 and a motor gear assembly including a second gear 52 disposed on the output shaft of the motor 51. In an initial state, the first gear 41 meshes with the second gear 52, and the motor 51 is configured to drive the transmission mechanism 6 to move to the distal end through the second gear 52, or to move to the proximal end through the first gear 41. When the first gear 41 is disengaged from the second gear 52, the connection mechanism 4 decouples from the electric mechanism 5, rendering the electric mechanism 5 incapable of driving the transmission mechanism 6. The output shaft of the motor 51, the axial direction of the second gear 52, and the axial direction of the first gear 41 are all parallel to a first direction. In alternative embodiments, the connection mechanism 4 may include multiple first gears 41, and/or, the motor gear assembly may comprise multiple second gears 52. One or more first gears 41 mesh with one or more second gears 52 so as to transmit power from the motor 51 to the transmission mechanism 6. Once those previously engaged gears are disengaged, the connection mechanism 4 and electric mechanism 5 become decoupled.

In the present application, the distal end and proximal end are defined relative to the operator, that is, one side closer to the operator is the proximal end, while another side farther from the operator (closer to the surgical site) is the distal end. An axial direction follows the longitudinal axis of the stapler, either from distal to proximal or proximal to distal. For example, in the perspective of Fig. 1, direction S1 represents a distal-to-proximal direction, and the transmission mechanism 6 may move along S1 or its opposite direction. Direction S2 in Fig. 1 is defined as a vertical direction (a height direction), while direction S3 represents a transverse direction (a width direction). For any component, the inner side indicates the portion closer to its center, and the outer side indicates the portion farther from its center.

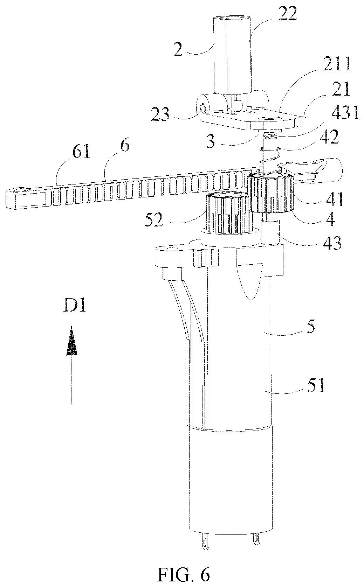

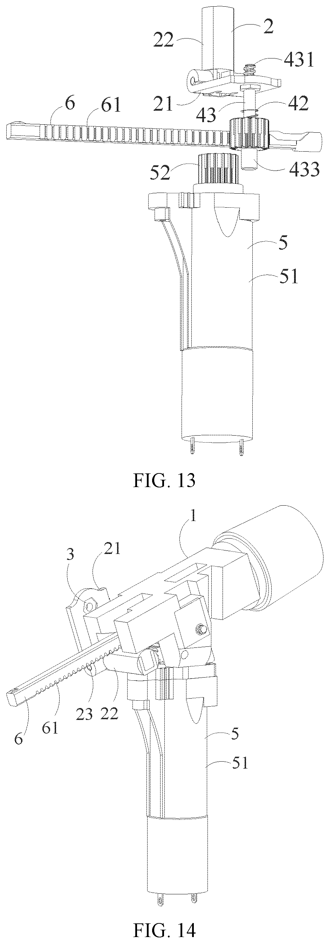

As shown in Figs. 1-8, the emergency apparatus comprises an outer frame 1, an inner frame 7 and a movable shaft 43. The outer frame 1 is sleeved over the first gear 41 and transmission mechanism 6, while the inner frame 7 is positioned inside the outer frame 1. The movable shaft 43 is movably disposed through the shaft hole 413 of the first gear 41. The movable shaft 43 and the first gear 41 could form two meshing states. When the movable shaft 43 and first gear 41 are located at a first relative position, the movable shaft 43 and first gear 41 are in the first meshing state (in a state of Fig. 6), the first gear 41 is rotatable around the movable shaft 43. When the movable shaft 43 and first gear 41 are located at a second relative position, the movable shaft 43 and first gear 41 are in the second meshing state (in a state of Fig. 13), the first gear 41 is prevented from rotating around the movable shaft 43, the first gear 41 is rotatable in response to the rotation of the movable shaft 43. While the movable shaft 43 moves along the first direction, the first gear 41 is driven to move along the same direction to be disengaged from the electric mechanism 5. Specifically, the second gear 52 is at least partially positioned on one side of the first gear 41 in the second direction, and the second direction opposites to the first direction. When the first gear 41 is driven by the movable shaft 43 to move along the first direction, the first gear 41 and the second gear 52 become out of meshing, thereby disengaging the first gear 41 from the electric mechanism 5.

In an event that an electric mechanism 5 fails to reset a transmission mechanism 6 after the firing of stapler, the manual reset applied to the transmission mechanism 6 could be achieved in two steps: Step 1: Driving the movable shaft 43 to move along the first direction with the first gear 41 remaining stationary, such that the movable shaft 43 and first gear 41 convert from the first relative position to the second relative position, thereby converting them from the first meshing state to the second meshing state, and continuously driving the movable shaft 43 to move along the first direction which drives the first gear 41 in the same direction to disengage from the electric mechanism 5 (being in a state as shown in Fig. 13). Step 2: driving the movable shaft 43 to rotate, thus driving the first gear 41 in its second meshing state to rotate, whereupon the first gear 41 cooperates with the teeth surface 61 of the transmission mechanism 6 to drive the transmission mechanism 6 to move toward the proximal end. In this embodiment, the first direction corresponds to upward direction D1 in Fig. 6, parallel to direction S2 in Fig. 1, and is perpendicular to the movement direction of the transmission mechanism 6 (direction S1 or its opposite direction).

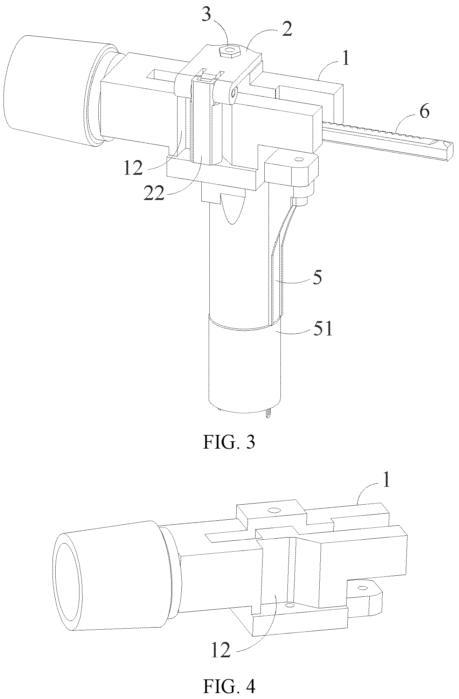

The movable shaft 43 is primarily driven in two modes: In the Step 1, the movable shaft 43 is driven to move linearly along the first direction and in the Step 2, the movable shaft 43 is driven to rotate. In this embodiment, the emergency apparatus further includes an operating mechanism 2 and a driving member 3, which are used for actuating the movable shaft 43 in these two modes. As shown in Fig. 2-6, the operating mechanism 2 includes a first operating lever 21 and a second operating lever 22. The driving member 3 is disposed at a first end of the first operating lever 21. The second end of the first operating lever 21 is pivotally connected to the second operating lever 22 via a pivot shaft 23. The operating mechanism 2 has two states: a storage state shown in Figs. 2-3 where the operating mechanism 2 is folded to be accommodated on one side of the outer frame 1 with the second operating lever 22 partially received in the receiving slot 12 of the outer frame 1 shown in Fig. 4. And an operational state where the driving member 3 located at the first operating lever 21 cooperates with the top portion of the movable shaft 43, enabling the operation performed by the users on the second operating lever 22 sequentially transmit an input, through the first operating lever 21 and driving member 3, to the movable shaft 43, so as to drive the movable shaft 43 to move. The inner frame 7 is provided with a limiting portion 71, sleeved over the top end of the movable shaft 43, located beneath the driving member 3. The limiting portion 71 limits a mating position between the first operating lever 21 and movable shaft 43. In this embodiment, the first end of the first operating lever 21 is provided with a mating hole 211, the driving member 3 is at partially embedded within the mating hole 211. The first operating lever 21 and the driving member 3 may form a fixed connection, or alternatively, the driving member 3 may be removable from the mating hole 211 when the driving member 3 is not in use. An outer surface of the driving member 3 cooperates with an inner surface of the mating hole 211 and a non-rotatable connection is formed therebetween. In this embodiment, these two surfaces are matching polygonal profiles. In alternative implementations, the driving member 3 and first operating lever 21 could be integrated, or be fixed together via fasteners.

As shown in Figs. 6-12, the movable shaft 43 includes a first threaded portion 431, a first mating portion 434, a second mating portion 432, and a third mating portion 433, which are sequentially arranged along the axial direction of the movable shaft 43. Both ends of the movable shaft 43 protrude beyond two end faces of the first gear 41, respectively, with the first threaded portion 431 disposed at the first end of the movable shaft 43 and the third mating portion 433 formed as a stepped portion at the second end thereof. The driving member 3 is provided with a second threaded portion 31. In this embodiment, the first threaded portion 431 includes an external thread while the second threaded portion 31 includes an internal thread. An outer diameter of the first mating portion 434 equals to or slightly exceeds the maximum outer diameter of the first threaded portion 431. The second threaded portion 31 and the first threaded portion 431 can form a threaded connection. When the second threaded portion 31 cooperates with the first threaded portion 431, the movable shaft 43 linearly moves along the first direction in response to the rotation of the driving member 3. In an event that the movable shaft 43 to a position where the first mating portion 434 cooperates with the second threaded portion 31, with these two form a non-rotatable connection, enabling the movable shaft 43 rotates in the same direction in response to the rotation of the driving member 3.

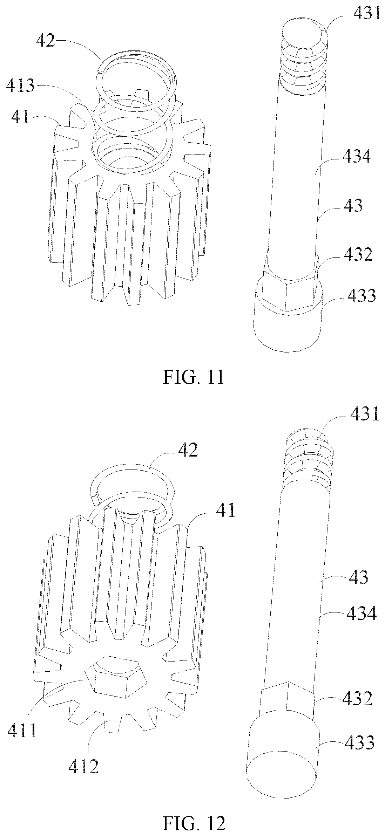

As shown in Figs. 11-12, the first mating portion 434 constitutes a plain shaft section of the movable shaft 43, while the second mating portion 432 is provided with a first anti-rotation structure on its outer surface. The first gear 41 includes a fourth mating portion 411 with a second anti-rotation structure and a fifth mating portion 412 formed by a bottom surface of the first gear 41. The first anti-rotation structure and the second anti-rotation structure are polygonal profiles matched correspondingly.

The outer diameter of the first mating portion 434 is less than the minimum size of the inner diameter of the fourth mating portion 411. Consequently, when the movable shaft 43 and first gear 41 are in the first meshing state, the first mating portion 434 cooperates with the fourth mating portion 411 to allow the first gear to rotate around the movable shaft 43. The outer diameter of the third mating portion 433 is greater than the inner diameter of the shaft hole 413 of the first gear 41. When the movable shaft 43 is driven to move along the first direction, the third mating portion 433 is prevented from entering the first gear 41, abuts against and cooperates with the fifth mating portion 412, so as to drive the first gear 41 to move along the first direction for disengaging the first gear 41 from the electric mechanism 5 without entering the first gear. When the first gear 41 disengages from the electric mechanism 5, the movable shaft 43 and first gear 41 are in the second meshing state where the second mating portion 432 cooperates with the fourth mating portion 411. The first anti-rotation structure cooperates with the second anti-rotation structure to prevent the first gear 41 from rotating around the movable shaft 43, enabling that the movable shaft 43 drives the first gear 41 to rotate in the same direction.

As shown in Figs. 11 and 12, the emergency apparatus further includes an elastic member 42, which is configured to apply a biasing force to the first gear 41 in a second direction opposite to the first direction. In this embodiment, the elastic member 42 includes a compression spring positioned above the first gear 41. In alternative implementations, the elastic member 42 may include other elastic configurations such as an elastic pad, an elastic sheet positioned above the first gear 41, or a tension spring positioned below the first gear 41.

The operational procedures of the emergency apparatus will be illustrated in detail with reference to Figs. 6 and 13.

In an initial state where the stapler has not been fired, the first gear 41 simultaneously meshes with both the second gear 52 and a rack of the transmission mechanism 6. The movable shaft 43 and first gear 41 are located at a first relative position where the first mating portion 434 of the movable shaft 43 cooperates with the fourth mating portion 411 of the first gear 41, and thus the movable shaft 43 and first gear 41 are in the first meshing state where the first gear 41 and the movable shaft 43 are relatively rotatable. During the firing of staple, the motor 51 is started to drive the first gear 41 to rotate by means of the second gear 52 to propel the transmission mechanism 6 to move to the distal end, thereby actuating the end effector to perform cutting-stapling functions. During the firing of the stapler, the movable shaft 43 and first gear 41 are in the first meshing state and are relatively rotatable, resulting that the movable shaft 43 is prevented from rotating.

After the firing of the stapler, the emergency apparatus represents a state as shown in Fig. 6, where the first gear 41 meshes with a teeth surface 61 of the transmission mechanism 6, the teeth surface 61 is located at the proximal end of the transmission mechanism 6, . In an event that the electric mechanism or a relating mechanic structure fails, requiring a manual reset of the transmission mechanism, the aforementioned Step 1 and Step 2 are adopted to reset the transmission mechanism. Specifically, In Step 1, firstly cooperating the second threaded portion 31 of the driving member 3 with the first threaded portion 431 located at the top end of the movable shaft 43, secondly operating the second operating lever 22 to rotate, transmitting such motion through the first operating lever 21 to the driving member 3, rotating the driving member 3, thereby moving the movable shaft 43 along the first direction, and the movable shaft 43 and the first gear 41 enters into the second relative position, where the second mating portion 432 of the movable shaft 43 enters the fourth mating portion 411 of the first gear 41 to form a relatively prohibited rotation therebetween, so that the movable shaft 43 and the first gear 41 enters the second meshing state. Simultaneously, the third mating portion 433 of the movable shaft 43 abuts against the fifth mating portion 412 of the first gear 41, the movable shaft 43 continues to move along the first direction, and the movable shaft 43 drives the first gear 41 to move along the same direction by such cooperation between the third mating portion 433 and the fifth mating portion 412. The first gear 41 disengages from the second gear 52 to disengage the connection mechanism 4 from the electric mechanism 5, while the elastic member 42 is compressed to form an elastic deformation. In this way, the emergency apparatus enters into a state as shown in Fig. 13. The elastic member 42 facilitates proper alignment of the movable shaft 43 with the first gear 41, enabling a smoother entry of the second mating portion 432 into the fourth mating portion 411.

After the connection mechanism 4 disengages from the electric mechanism 5, the second threaded portion 31 cooperates with the first mating portion 434 of the movable shaft 43. Accordingly, the length of the first threaded portion 431 equals to a stroke of the movable shaft 43 from its initial position to a position where the first gear 41 disengages from the second gear 52. Step 2 is performed at this moment: continuously rotating the second operating lever 22 to rotate the second threaded portion 31 through the first operating lever 21, the second threaded portion 31 drives, through movable shaft 43, the first gear 41 to rotate in the same direction, and subsequently the first gear 41 drives the transmission mechanism 6 engaging with the first gear 41 to move along a axial direction of the stapler to the proximal end, thereby achieving a reset of the transmission mechanism 6. The emergency apparatus represents a simple structure with convenient operation, effectively enabling manual reset of the transmission mechanism 6 in an event that the electric mechanism 5 fails to reset.

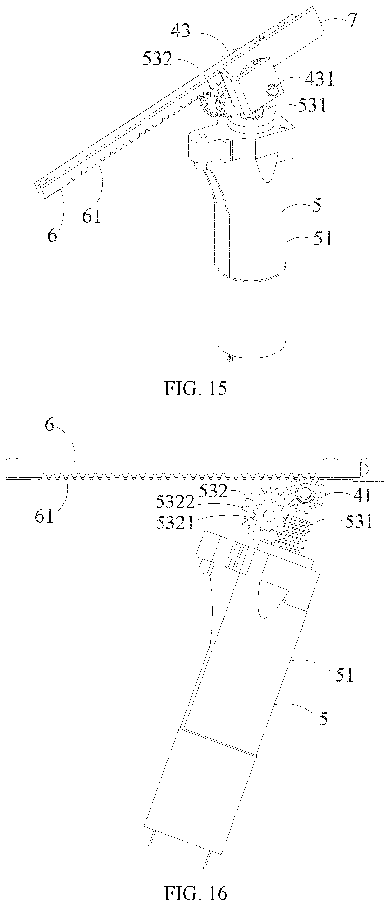

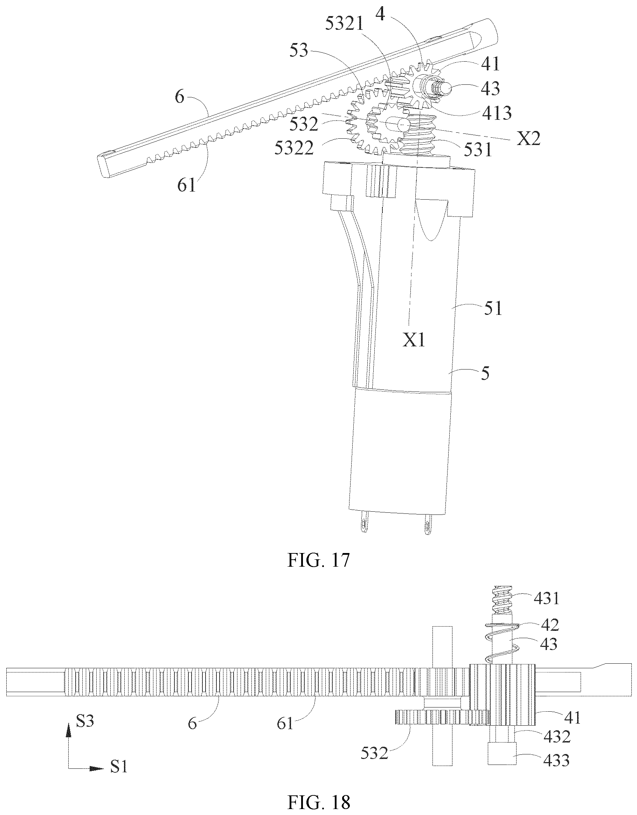

Figs. 14-20 illustrate a mating structure between the emergency apparatus and other instrument components in the second embodiment of the present application. This embodiment differs from the first embodiment primarily in that the operating mechanism 2 in the first embodiment drives the movable shaft 43 to move on a top side of the outer frame 1, and the operating mechanism 2 in the second embodiment drives the movable shaft 43 to move on one lateral side of the outer frame 1. As shown in Fig. 15, the inner frame 7 serves to support and fix the first gear 41 and movable shaft 43, and the movable shaft 43 is disposed through the inner frame 7 such that the first threaded portion 431 remains exposed for cooperating with the driving member 3 of the operating mechanism 2. Figs. 16-18 illustrate that in this embodiment, the motor gear assembly 53 includes a first motor gear 531 and a second motor gear 532 that have axes arranged crossly. Fig. 17 specifically illustrates an axis X1 of the first motor gear 531 (aligned with the output shaft of motor 51) and an axis X2 of the second motor gear 532. It can be seen from Fig. 17 that X1 and X2 intersect each other. The first motor gear 531 is disposed over the output shaft of the motor, which maintains an angular offset relative to the first direction. The second motor gear 532 incorporates a first teeth portion 5321 and second teeth portion 5322. In the initial state, the first teeth portion 5321 meshes with the first motor gear 531, while the second teeth portion 5322 meshes with the first gear 41. When the motor 51 is started, the motor 51 drives the first gear 41 to rotate sequentially through the first motor gear 531 and second motor gear 532.

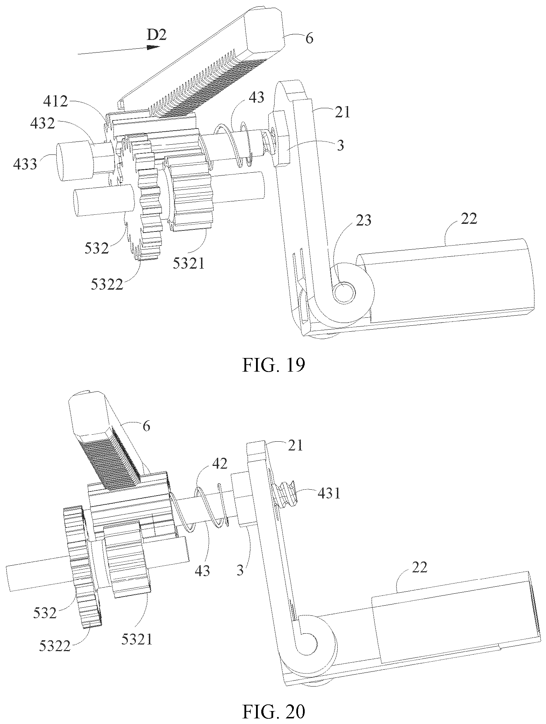

As shown in Figs. 18-20, the first direction D2 (parallel to a direction S3 in Fig. 18) is perpendicular to the movement direction of the transmission mechanism 6 (a direction S1 in Fig. 1). In the initial state, the second teeth portion 5322 of the second motor gear 532 meshes with the first gear 41. In a state as shown in Fig. 18, rotation of the second operating lever 22 drives the movable shaft 43 to move along the first direction D2, which in turn drives the first gear 41 to move in the same direction to disengage from the second teeth portion 5322 of the second motor gear 532, achieving the state illustrated in Fig. 19. The first teeth portion 5321 has an outer diameter smaller than that of the second teeth portion 5322, thereby preventing interference with the first gear 41. With the movable shaft 43 and the first gear 41 now in the second meshing state, the transmission mechanism 6 may be reset by driving the movable shaft 43 and the first gear 41 to rotate through the driving member 3.

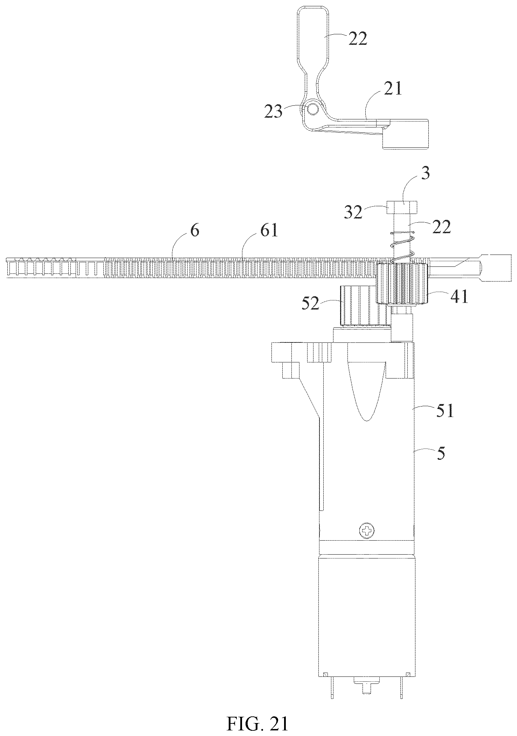

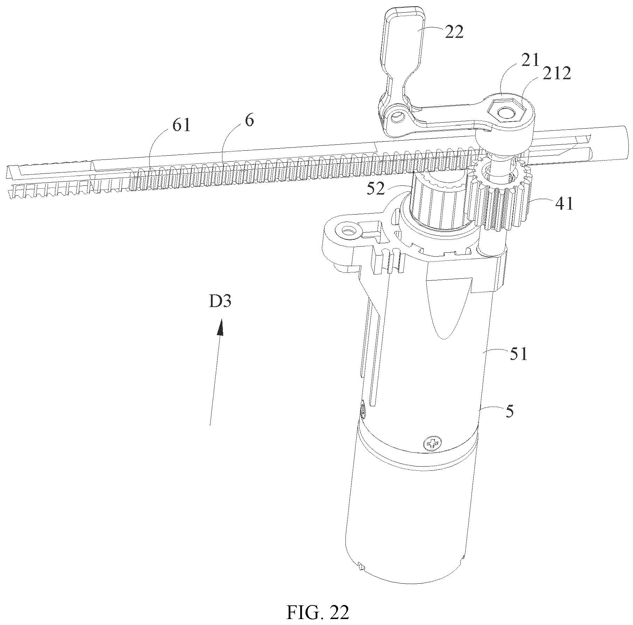

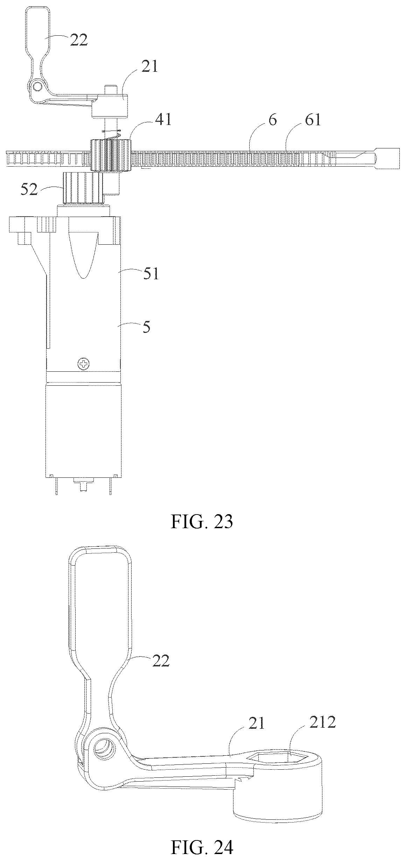

Figs. 21-24 illustrate the structural cooperation between the emergency apparatus and other instrument components according to the third embodiment of the present application. This embodiment differs from the first embodiment primarily in that the driving member 3 is detachably connected to the first end of the first operating lever 21. The first end of the first operating lever 21 is provided with a mating groove 212, and the driving member 3 is at least partially embedded. As shown in Fig. 24, the outer surface of the driving member 3 serves as a mating surface 32 that mates with the inner surface of the mating groove 212 to form a non-rotatable connection. In this embodiment, both the mating surface 32 of the driving member 3 and the inner surface of the mating groove 212 are polygonal surfaces. When stowing the operating mechanism 2 during non-use, the driving member 3 may remain engaged with the top end of the movable shaft 43, maintaining the threaded connection between its internal second threaded portion and the first threaded portion 431. In the initial state, the second gear 52 meshes with the first gear 41. In a configuration as shown in Fig. 22, rotation of the second operating lever 22 drives the movable shaft 43 along the first direction D3, consequently moving the first gear 41 in the same direction to disengage from the second gear 52 and achieve the state illustrated in Fig. 23. With the movable shaft 43 and the first gear 41 now in the second meshing state, the transmission mechanism 6 may be reset by driving the movable shaft 43 and the first gear 41 to rotate through the driving member 3.

The above description with reference to specific preferred embodiments provides further detailed explanation of the present application, but should not be construed as limiting the implementation of the present application solely to these illustrations. For those skilled in the art to which the present application pertains, various simple derivations or substitutions may be made without departing from the inventive concept of the present application, and all such modifications shall be considered within the protection scope of the present application.

## Claims

1. An emergency apparatus for a surgical instrument, wherein the surgical instrument comprises: a transmission mechanism (6), driven to move toward or away from an end effector of the surgical instrument; a connection mechanism (4), connected to the transmission mechanism (6); and an electric mechanism (5), connected to the connection mechanism (4) and configured to drive the connection mechanism (4) to move, thereby driving the transmission mechanism (6) to move, wherein the connection mechanism (4) comprises a first gear (41), the connection mechanism (4) is capable of disengaging from the electric mechanism (5), wherein, the emergency apparatus comprises:
a movable shaft (43) movably disposed through the first gear (41), wherein when the movable shaft (43) and the first gear (41) are in a first meshing state, the first gear (41) is rotatable around the movable shaft (43); when the movable shaft (43) and the first gear (41) are in a second meshing state, the first gear (41) is prevented from rotating around the movable shaft (43) and is configured to move in response to a movement of the movable shaft (43) so as to disengage from the electric mechanism (5), wherein a movement direction of the movable shaft (43) is consistent with a movement direction of the first gear (41).

2. The emergency apparatus according to claim 1, wherein
when the movable shaft (43) and the first gear (41) are located at a first relative position, the movable shaft (43) and the first gear (41) are in the first meshing state;
when the movable shaft (43) and the first gear (41) are located at a second relative position, the movable shaft (43) and the first gear (41) are in the second meshing state;
wherein the movable shaft (43) and the first gear (41) are capable of converting from the first relative position to the second relative position in response to said movement of the movable shaft (43) in a first direction, and the first direction is perpendicular to a direction in which the transmission mechanism (6) moves toward or away from the end effector of the surgical instrument.

3. The emergency apparatus according to claim 2, wherein
the movable shaft (43) comprises a first mating portion (434), a second mating portion (432) and a third mating portion (433); the first gear (41) comprises a fourth mating portion (411) and a fifth mating portion (412);
when the movable shaft (43) and the first gear (41) are in the first meshing state, the first mating portion (434) cooperates with the fourth mating portion (411) to allow the first gear (41) to rotate around the movable shaft (43);
when the movable shaft (43) and the first gear (41) are in the second meshing state, the second mating portion (432) cooperates with the fourth mating portion (411) to prevent the first gear (41) from rotating around the movable shaft (43);
when the movable shaft (43) is driven to move, the third mating portion (433) cooperates with the fifth mating portion (412) to drive the first gear (41) to move, thereby disengaging the first gear (41) from the electric mechanism (5).

4. The emergency apparatus according to claim 3, wherein the first gear (41) comprises a shaft hole (413), the movable shaft (43) is disposed through the shaft hole (413) of the first gear (41), and the movable shaft (43) is provided with a first threaded portion (431);
the emergency apparatus further comprises a driving member (3) provided with a second threaded portion (31) cooperating with the first threaded portion (431), wherein the movable shaft (43) is configured to move in response to a rotation of the driving member (3).

5. The emergency apparatus according to claim 4, wherein both ends of the movable shaft (43) protrude beyond two end faces of the first gear (41), respectively, the first threaded portion (431) is disposed at a first end of the movable shaft (43), the third mating portion (433) is disposed at a second end of the movable shaft (43), and an outer diameter of the third mating portion (433) is greater than an inner diameter of the shaft hole (413) of the first gear (41).

6. The emergency apparatus according to claim 5, wherein the first mating portion (434) of the movable shaft (43) is located between the first threaded portion (431) and the second mating portion (432), and an outer diameter of the first mating portion (434) is less than or equal to the inner diameter of the shaft hole (413) of the first gear (41).

7. The emergency apparatus according to claim 6, wherein, after the first gear (41) is disengaged from the electric mechanism (5), when the movable shaft (43) is driven to rotate by said rotation of the driving member (3), the first gear (41) is configured to rotate in response to a cooperation between the second mating portion (432) and the fourth mating portion (411).

8. The emergency apparatus according to claim 4, wherein the emergency apparatus further comprises an elastic member (42) configured to apply a biasing force to the first gear (41) in a second direction opposite to the first direction.

9. The emergency apparatus according to claim 4, wherein the first threaded portion (431) includes an external thread distributed on an outer surface of the movable shaft (43), the driving member (3) is at least partially sleeved over the first threaded portion (431); the second threaded portion (31) includes an internal thread distributed on an inner surface of the driving member (3).

10. The emergency apparatus according to claim 9, wherein the emergency apparatus further comprises a first operating lever (21), wherein the driving member (3) is provided at a first end of the first operating lever (21).

11. The emergency apparatus according to claim 10, wherein the first end of the first operating lever (21) is provided with a mating hole (211) or a mating groove (212), and the driving member (3) is at least partially embedded within the mating hole (211) or the mating groove (212).

12. The emergency apparatus according to claim 11, wherein an outer surface of the driving member (3) cooperates with an inner surface of the mating hole (211) or the mating groove (212) to form a non-rotatable connection.

13. The emergency apparatus according to claim 11, wherein the driving member (3) is detachably connected to the first end of the first operating lever (21).

14. The emergency apparatus according to claim 10, wherein the emergency apparatus further comprises a second operating lever (22) connected to a second end of the first operating lever (21).

15. The emergency apparatus according to claim 14, wherein the emergency apparatus further comprises an outer frame (1), wherein the first gear (41) and the movable shaft (43) are both at least partially located inside the outer frame (1); one side of the outer frame (1) is provided with a receiving groove (12), and the second operating lever (22) is at least partially disposed within the receiving groove (12).

16. The emergency apparatus according to claim 4, wherein the electric mechanism (5) comprises a motor (51) and a motor gear assembly (53), wherein an output shaft of the motor (51) is configured to drive the motor gear assembly (53) to rotate, the motor gear assembly (53) is at least partially located on one side of the first gear (41) in a second direction opposite to the first direction, the motor gear assembly (53) comprises a second gear (52), and when the electric mechanism (5) is connected to the connection mechanism (4), the first gear (41) meshes with the second gear (52) with the output shaft of the motor (51) being parallel to the first direction.

17. The emergency apparatus according to claim 16, wherein the motor gear assembly (53) comprises a first motor gear (531) and a second motor gear (532), the first motor gear (531) and the second motor gear (532) have axes arranged crossly, wherein the output shaft of the motor (51) is parallel to the first direction or forms at an angle with the first direction.

18. A surgical instrument, **characterized by** comprising the emergency apparatus according to any one of claims 1 to 17.
